# EUROPEAN PATENT SPECIFICATION

(11) **EP 4 370 093 B1**
(45) Date of publication and mention of the grant of the patent: **30.07.2025**
(21) Application number: 22722264.3
(22) Date of filing: 12.04.2022
(51) Int. Cl.: A61K 9/00, A61K 9/08, A61K 47/10, A61K 47/44, A61K 47/26, A61M 15/00, A61M 15/08, A61P 11/00, A61K 31/196

(54) **AQUEOUS BUMETANIDE-CONTAINING LIQUID**
WÄSSRIGE BUMETANIDHALTIGE FLÜSSIGKEIT
LIQUIDE AQUEUX CONTENANT DE LA BUMÉTANIDE

(30) Priority: 16.07.2021 EP 21186148
(43) Date of publication of application: 22.05.2024
(73) Proprietor: DriPel B.V., 3572 CA Utrecht (NL)
(72) Inventor: PELLIKAAN, Hubert Clemens, 3572 CA Utrecht (NL); VAN DRIEL, Vincent Jean Hendrik Michel, 3735 MX Bosch en Duin (NL)
(74) Representative: Nederlandsch Octrooibureau
(86) International application number: PCT/EP2022/059741
(87) International publication number: WO 2023/285009

(56) References cited:
- EP-A1- 0 139 127
- WO-A1-2021/113775
- NIELSEN H ET AL: "Intranasal administration of different liquid formulations of bumetanide to rabbits", INTERNATIONAL JOURNAL OF PHARMACEUTICS, ELSEVIER, NL, vol. 204, no. 1-2, 20 June 2000 (2000-06-20), pages 35 - 41, XP027379985, ISSN: 0378-5173, [retrieved on 20000620]

## Description

### TECHNICAL FIELD OF THE INVENTION

The present invention relates to an aqueous liquid comprising: 0.3-5 wt.% bumetanide; 2-30 wt.% of a non-ionic surfactant having a HLB of more than 10; and 65-94 wt.% water, said liquid having a pH at 25°C in the range of 5-9 and the bumetanide and the non-ionic surfactant being dissolved and/or contained in colloidal particles having a diameter of less than 50 nm.

The invention further relates to a spraying device comprising a container holding said aqueous bumetanide-containing liquid and to the use of the aqueous bumetanide-containing liquid in medical treatment comprising transmucosal administration of the liquid.

### BACKGROUND OF THE INVENTION

Bumetanide is a medication used to treat edema caused by conditions such as heart failure, liver failure, or kidney problems. Administration of bumetanide can lessen symptoms such as shortness of breath and swelling in arms, legs, and abdomen. Bumetanide is a diuretic that causes patients to produce more urine and to get rid of (extra) water and salt. Bumetanide is taken orally, or by injection into a vein or muscle. Effects generally begin within an hour and lasts for about six hours. Bumetanide has a very low water solubility (26 mg/L at 25°C). Bumetanide is a diprotic acid with a pKₐ₁ of 3.6 and a Pkₐ₂ of 7.7.

Transmucosal administration of drugs has the potential to improve therapy efficacy by increasing patient's treatment adherence and reducing side effects compared to, for instance, intravenous and oral administration.

In the past century, the use of intranasally administered drugs was mainly restricted to treating topical symptoms of seasonal rhinitis or infectious diseases of the respiratory tract, for example. It was only at the end of the twentieth century that nasal delivery became more prominent as an alternative route for systemic therapy. The surface of the nasal mucosa in humans is around 150 cm². This tissue is well supplied by blood vessels in extreme close proximity to the mucosal surface (blood vessels and mucosal surface are separated by only one mucosal cell layer), which allows for a rapid absorption and high systemic blood levels of the drug, while avoiding the first pass metabolism.

Despite the increasing number of approved nasal formulations for transmucosal absorption, the development of new formulations remains challenging, especially for formulations based on hydrophobic drugs, i.e. drugs having a very low water solubility. This is because (i) water is the preferred solvent, (ii) the amount of formulation that can be administered intranasally is limited and (iii) many pharmaceutical excipients cause irritation.

WO 2021/113775 describes compositions for intranasal, sublingual, and subcutaneous administration of bumetanide for the treatment of subjects suffering from edema refractory to oral diuretics. The examples describe compositions comprising bumetanide salt and emulsions comprising bumetanide. Example 1 describes an experiment in which the effect of non-ionic surfactant (tetradecyl-beta-D-maltoside) on water-solubility was investigated.

Nielsen, Hanne W., et al. (Solubilization and stability of bumetanide in vehicles for intranasal administration, a pilot study. Pharmaceutical development and technology 6.2 (2001): 145-149) describe the solubility of bumetanide in vehicles of various polarities, suitable for intranasal administration in acute situations. The solubility at 4°C in glycofurol and polyethylene glycol 200 was 167 and 143 mg/mL, respectively, decreasing exponentially with addition of phosphate buffer or coconut oil.

Nielsen, H. W., et al. (Intranasal administration of different liquid formulations of bumetanide to rabbits. International journal of pharmaceutics 204.1-2 (2000): 35-41) describe the bioavailability of bumetanide in rabbits after intranasal administration of eight formulations intended for use in acute situations. The vehicles tested were combinations of phosphate buffer, pH 7.4, glycofurol 75, polyethylene glycol 200 and coconut oil.

### SUMMARY OF THE INVENTION

The inventors have developed an aqueous bumetanide-containing liquid that can suitably be used for transmucosal administration of bumetanide, said aqueous liquid comprising:
- 0.3-5 wt.% bumetanide;
- 2-30 wt.% of a non-ionic surfactant having a HLB of more than 10; and
- 65-94 wt.% water;
wherein the liquid has a pH at 25°C in the range of 5-9 and wherein each of the bumetanide and the non-ionic surfactant is dissolved and/or contained in colloidal particles having a diameter of less than 50 nm.

The aqueous liquid of the present invention offers the advantage that it can accommodate relatively high concentrations of bumetanide. In comparison to e.g. emulsions, the aqueous liquid has excellent storage stability across a broad temperature range. Furthermore, the liquid is easy to manufacture and enables uniform and accurate dosing. The aqueous liquid also offers the convenience that it can be administered transmucosally without causing irritation, e.g. stinging or itching.

Excellent bioavailability can be achieved upon transmucosal administration of the aqueous bumetanide-containing liquid of the present invention. Although the inventors do not wish to be bound by theory it is believed that the presence of a high amount of nonionic surfactant having a HLB of more than 10 not only aids the dissolution of bumetanide salt, but also enhances the uptake of bumetanide through the mucosa.

Transmucosal administration of bumetanide represents an advantageous solution for patients experiencing impaired gastrointestinal absorption due to the excess of fluid. This scenario normally requires higher dosage of oral diuretics, which in turn further increases the strain on the kidneys and liver.

The invention also provides a spraying device comprising:
- a container holding the aqueous bumetanide-containing liquid of the present invention,
- a nozzle in fluid connection with the container, and
- a metered dose pump configured to create a flow of the aqueous bumetanide-containing liquid through the nozzle upon activation.

Another aspect of the invention relates to the administration of the aqueous bumetanide-containing liquid of the present invention to prevent or treat edema, hyperkalemia, epileptic seizures, autism, hypertension, renal insufficiency, nephrotic syndrome, liver cirrhosis and/or heart failure.

### DETAILED DESCRIPTION OF THE INVENTION

A first aspect of the invention relates to an aqueous bumetanide-containing liquid comprising:
- 0.3-5 wt.% bumetanide;
- 2-30 wt.% of a non-ionic surfactant having a HLB of more than 10; and
- 65-94 wt.% water;
wherein the liquid has a pH at 25°C in the range of 5-9 and wherein each of the bumetanide and the non-ionic surfactant is dissolved and/or contained in colloidal particles having a diameter of less than 50 nm.

The term HLB is an abbreviation of "hydrophile-lipophile balance". The hydrophilic-lipophilic balance of a surfactant is a measure of the degree to which it is hydrophilic or lipophilic. The HLB as referred to in here is determined as described by J.T. Davies (A quantitative kinetic theory of emulsion type. I. physical chemistry of the emulsifying agent, Gas/Liquid and Liquid/liquid interfaces, Proceedings of 2nd International Congress Surface Activity, Butterworths, London 1957).

The diameter of colloidal particles can suitably be determined as the scattering intensity-weighted mean diameter, z-average, measured at 25 °C by dynamic light scattering (Zetasizer Nano-ZS, Malvern Instruments).

The aqueous bumetanide-containing liquid of the present invention preferably comprises 0.5-4 wt.%, more preferably 0.6-3 wt.%, even more preferably, 0.8-2.5 wt.%, most preferably 1.0-2.0 wt.% of bumetanide.

In a preferred embodiment, the non-ionic surfactant contained in the aqueous bumetanide-containing liquid of the present invention has a HLB of at least 12, more preferably of at least 13, most preferably of at least 14.

The HLB of the ionic surfactant preferably does not exceed 24, more preferably it does not exceed 22, most preferably it does not exceed 20.

The non-ionic surfactant is preferably contained in the aqueous bumetanide-containing liquid in a concentration of at least 4 wt.%, more preferably of at least 5 wt.%, even more preferably of at least 6 wt.%, yet more preferably of at least 7.5 wt.%, and most preferably of at least 9 wt.%.

In a preferred embodiment, the non-ionic surfactant is present in the aqueous liquid in a concentration that does not exceed 30 wt.%, more preferably does not exceed 25 wt.%, most preferably does not exceed 20 wt.%.

Preferably the non-ionic surfactant is selected from polyoxyethylene sorbitan esters of fatty acids, glycerol polyethylene glycol hydroxyl-fatty acid, polyethyleneglycol esters of fatty acids, poloxamers and mixtures thereof. More preferably, the non-ionic surfactant is selected from polyoxyethylene sorbitan esters of fatty acids. Most preferably, the non-ionic surfactant is polyoxyethylene (20) sorbitan monooleate.

Commercially available examples of suitable non-ionic surfactants according to the invention include Cremophor RH 40, Cremophor EL, Tween 20, Tween 80 and Pluronic F127.

In a further preferred embodiment, the aqueous liquid comprises at least 5 wt.%, more preferably at least 7.5 wt.%, most preferably at least 9 wt.% polyoxyethylene (20) sorbitan monooleate.

The aqueous bumetanide-containing liquid of the present invention preferably has a pH at 25°C in the range of 6 to 8, more preferably in the range of 6.5 to 7.5.

The aqueous bumetanide-containing liquid preferably comprises a buffer having a buffering pH in the range of 6 to 8. The buffer is preferably selected from phosphate buffer, ammonium acetate buffer and citrate buffer.

According to a preferred embodiment the aqueous bumetanide-containing liquid contains ammonium, sodium, potassium, lithium and/or arginine in a combined molar concentration that exceeds 50%, more preferably exceeds 80% of the molar concentration of bumetanide.

According to a preferred embodiment the aqueous bumetanide-containing liquid contains sodium, potassium and/or arginine in a combined molar concentration that exceeds 50%, more preferably exceeds 80% of the molar concentration of bumetanide. More preferably, the liquid contains sodium and/or potassium in a combined molar concentration that exceeds 50%, more preferably exceeds 80% of the molar concentration of bumetanide. Most preferably, the liquid contains sodium in a molar concentration that exceeds 50%, more preferably exceeds 80% of the molar concentration of bumetanide.

According to another, particularly preferred embodiment, the aqueous bumetanide-containing liquid contains ammonium in a molar concentration that exceeds 50%, more preferably exceeds 80% of the molar concentration of bumetanide.

According to another preferred embodiment, the aqueous bumetanide-containing liquid contains arginine and/or potassium in a combined molar concentration that does not exceed 30%, preferably 20%, more preferably 15% of the molar concentration of bumetanide.

In an embodiment wherein the aqueous bumetanide-containing liquid contains sodium in a molar concentration that exceeds 50% of the molar concentration of bumetanide, bumetanide is preferably contained in the liquid in a concentration of at least 0.6 wt.%, more preferably of at least 0.7 wt.%, most preferably of at least 0.8 wt.%.

In an embodiment wherein the aqueous bumetanide-containing liquid contains potassium in a molar concentration that exceeds 50% of the molar concentration of bumetanide, bumetanide is preferably contained in the liquid in a concentration of at least 2.2 wt.%, more preferably of at least 2.5 wt.%, most preferably of at least 3 wt.%.

In an embodiment wherein the aqueous bumetanide-containing liquid contains arginine in a molar concentration that exceeds 50% of the molar concentration of bumetanide, bumetanide is preferably contained in the liquid in a concentration of at least 1.4 wt.%, more preferably of at least 1.5 wt.%., most preferably of at least 1.8 wt.%

In an embodiment wherein the aqueous bumetanide-containing liquid contains ammonium in a molar concentration that exceeds 50% of the molar concentration of bumetanide, bumetanide is preferably contained in the liquid in a concentration of at least 1.0 wt.%, more preferably of at least 1.8 wt.%, most preferably of at least 2.2 wt.%.

The aqueous bumetanide-containing liquid of the present invention may suitably contain a cosolvent. Examples of co-solvents that may be employed include ethanol, isopropyl alcohol, glycerol, propylene glycol, polyethylene glycol, glycofurol and combinations thereof.

Preferably the combination of water and the non-ionic surfactant constitutes at least 80 wt.%, more preferably at least 90 wt.% and most preferably at least 95 wt.% of the aqueous bumetanide-containing liquid.

Preferably the aqueous bumetanide-containing solution is transparent. Transparency offers the advantage that users can visually ascertain that no microbial growth or precipitation of bumetanide has occurred.

According to a preferred embodiment, bumetanide is fully dissolved in the aqueous liquid of the invention.

Preferably, the non-ionic surfactant is fully dissolved in the aqueous liquid.

The aqueous bumetanide-containing liquid of the present invention preferably is an isotonic solution.

According to a preferred embodiment, the aqueous bumetanide-containing liquid does not comprise cationic and/or anionic surfactants.

Preferably, the aqueous bumetanide-containing liquid does not comprise phospholipids.

In accordance with another preferred embodiment, the aqueous bumetanide-containing liquid does not comprise cyclodextrin.

In a preferred embodiment, the aqueous bumetanide-containing liquid has a viscosity at 20°C and a shear rate of 16 s⁻¹ of not more than 200 mPa.s, preferably less than 100 mPa.s, more preferably 2-60 mPa.s measured with a Brookfield viscometer model RVDVII+ and using spindle 3.

The viscosity of the aqueous bumetanide-containing liquid may be increased by adding a suitable thickening agent. Examples of thickening agents that may suitably be applied include alginic acid, alginate, HPMC, pectin, microcrystalline cellulose, carboxymethyl cellulose, gellan, carrageenan, xanthan gum, agar, vegetable gums and combinations thereof. Typically, thickening agent is incorporated in a concentration of 0.1-5 wt.%, more preferably in a concentration of 0.15-3 wt.%.

The aqueous bumetanide-containing liquid of the invention preferably is a sterile composition. Sterilization may be achieved by methods known in the art, such as heat sterilization, high pressure sterilization and filtration sterilization.

The aqueous bumetanide-containing liquid may further comprise an effective amount of a preservative. Suitable preservatives are, for instance, phenoxy ethanol, benzalkonium chloride and benzyl alcohol, EDTA, potassium sorbate, parahydroxybenzoate and combinations thereof.

The aqueous bumetanide-containing liquid may comprise antioxidants. Suitable antioxidants are for example ascorbic acid, carotenoids, vitamin A and tocopherol.

Yet another aspect of the invention relates to a spraying device comprising
- a container holding the aqueous bumetanide-containing liquid as defined herein before,
- a nozzle in fluid connection with the container, and
- a metered dose pump configured to create a flow of the aqueous bumetanide-containing liquid through the nozzle upon activation.

A specific type of spraying device has a microfilter in the air inlet, so the bottle cannot be contaminated in-use by incoming air. Another specific type of spraying device has no incoming air inlet and also has a spraying nozzle that is hermetically sealed at the end of each spray so no air can enter the device. This enables the oxygen free operation of the device. In case the product is prepared oxygen free, it will stay oxygen free during its entire use.

The spraying device of the present invention is preferably configured to deliver one spray dose upon nozzle activation. The activation of said nozzle is preferably a manual activation. Said spray dose preferably has a volume of 50 to 300 µl, more preferably of 70 to 200 µl.

Another aspect of the invention relates to the use of the aqueous bumetanide-containing liquid as defined herein before in medical treatment, said treatment preferably comprising transmucosal administration of the liquid.

Examples of transmucosal administration include intranasal, intra-oral and intra-ocular administration. In a particularly preferred embodiment of the aforementioned treatment the aqueous bumetanide-containing liquid is administered intranasally.

More preferably, said the aqueous bumetanide-containing liquid is used in the prevention or treatment of edema, hyperkalemia, epileptic seizures, autism, hypertension, renal insufficiency, nephrotic syndrome, liver cirrhosis and/or heart failure, wherein the liquid is administered transmucosally.

In a preferred embodiment the aqueous bumetanide-containing liquid is administered transmucosally to deliver bumetanide in a dose of 0.5 to 5 mg, more preferably in a dose of 0.8-3 mg and most preferably in a dose of 1-2 mg. Preferably, the administration comprises 1-3 spray doses, said spray doses preferably having a volume of 50-300 µl.

Preferably the aqueous bumetanide-containing liquid is used in the treatment of a human.

The invention is further illustrated by the following non-limiting examples.

### EXAMPLES

### Example 1

Formulations were prepared on the basis of the recipes shown in Table 1:

**Table 1**

| | **Wt. %** | | | |
|---|---|---|---|---|
| | **A** | **1** | **2** | **3** |
| Bumetanide | 0.7 | 0.7 | 1.11 | 2.4 |
| Polysorbate 80 | 0.0 | 8.0 | 12.1 | 30 |
| NaOH [10%] | q. s. to pH 7.0 | q. s. to pH 7.0 | q. s. to pH 7.0 | q. s. to pH 7.0 |
| Aqua purificata | Ad 100 | Ad 100 | Ad 100 | Ad 100 |

The formulations were prepared as follows. The surfactant is placed in a beaker, the water and an equimolar amount of NaOH (relative to the Bumetanide) are added, followed by mixing using a magnetic stirrer until a clear and homogeneous solution is formed. To this the bumetanide (in acid form) is added. The pH is adjusted to 7.0 using NaOH (10% solution). In this way, clear liquids were obtained for formulations 1, 2 and 3.

The bumetanide in formulation A, however, could not be fully dissolved. Complete dissolution could only be achieved by heating the mixture to 70°C. However, upon cooling down to ambient temperature, formulation A formed a precipitate.

Formulations 1, 2 and 3 were stored at 4°C for 7 days. No precipitation was observed during this period..

The above mentioned formulations were intranasally administered to human volunteers using a spraying device that delivered a dose of approximately 100 µl. The results are summarized in Table 2.

**Table 2**

| | **A** | **1** | **2** | **3** |
|---|---|---|---|---|
| sprayability | excellent | excellent | excellent | OK |
| Leakage | No leakage | No leakage | No leakage | No leakage |
| Irritation | none | none | none | none |

### Example 2

The diuretic effect of an intranasal formulation according to the present invention was compared with the diuretic effect of a 1 mg bumetanide tablet and the diuretic effect of a placebo tablet (control). Since the diuretic effect was tested in healthy volunteers (without edema) the volunteers were asked to replenish their loss of urine by drinking the same amount of aqueous beverage.

An intranasal formulation was prepared in the same way as in Example 1 (buffer was added before pH adjustment). The composition of the intranasal formulation is shown in Table 3.

**Table 3**

| | **Wt.%** |
|---|---|
| Bumetanide | 1.0 |
| Polysorbate 80 ¹ | 11 |
| NaHPO₄ | 0.5 |
| NaOH [10%] | q. s. to pH 7.0 |
| Aqua purificata | Ad 100 |

| | |
|---|---|
| ¹ HLB = 15 | |

The intranasal formulation was administered by means of a spraying device that delivered a dose of approximately 100 µl (1 mg bumetanide). In order to administer 2 mg bumetanide a single dose of the formulation was administered in each nostril. The results of the test are summarized in Table 4.

**Table 4**

| | **Cumulative urine production (in ml)** | | | |
|---|---|---|---|---|
| **Time period after administration** | **Control** | **1 mg oral** | **1 mg intrana- sal** | **2 mg intrana-sal** |
| 1 hour | 41 | 265 | 63 | 325 |
| 2 hours | 232 | 680 | 613 | 1150 |
| 3 hours | 317 | 1240 | 1220 | 1860 |
| 4 hours | 401 | 1740 | 1620 | 2280 |
| 5 hours | 496 | 1970 | 1690 | 2480 |
| 6 hours | 562 | 1970 | 1820 | 2530 |

### Example 3

Intranasal formulations containing sodium bumetanide were prepared in the same as in Example 2 on the basis of the recipes shown in Table 5.

**Table 5**

| | **Wt.%** | | | |
|---|---|---|---|---|
| | **1** | **2** | **3** | **4** |
| Bumetanide | 1.11 | 1.11 | 1.11 | 1.11 |
| Polysorbate 80 ¹ | 12.1 | | | |
| Polysorbate 20 ² | | 12.1 | | |
| Polyethoxylated castor oil ³ | | | 12.1 | |
| Poloxamer ⁴ | | | | 17.0 |
| NaOH [10%] | q. s. to pH 7.0 | | | |
| Aqua purificata | Ad 100 | | | |

| | | | | |
|---|---|---|---|---|
| ¹ HLB = 15 ² HLB = 16.7 ³ Cremophor-EL, HLB = 13.5 ⁴ Pluronic F-127, HLB = 18 | | | | |

The stability of these formulations at storage temperatures of 25°C and 4°C was tested. It was found that all four formulations were completely stable at these temperature, i.e. no precipitation was observed.

### Example 4

Intranasal formulations containing different bumetanide salts were prepared in the same as in Example 2 on the basis of the recipes shown in Table 6.

**Table 6**

| | **Wt. %** | | | |
|---|---|---|---|---|
| | **1** | **2** | **3** | **4** |
| Bumetanide | 1.11 | 2.4 | 1.11 | 2.4 |
| Polysorbate 80 | 12.1 | 30.0 | 12.1 | 30 |
| KOH [10%] | q. s. to pH 7.0 | | 0 | 0 |
| Arginine [10%] | 0 | 0 | q. s. to pH 7.0 | |
| Aqua purificata | Ad 100 | | | |

The stability of these formulations at storage temperatures of 25°C and 4°C was tested. It was found that all four formulations were completely stable at these temperature, i.e. no precipitation was observed.

### Comparative Example A

Intranasal formulations were prepared in the same as in Example 2 on the basis of the recipes shown in Table 7.

**Table 7**

| | **Wt.%** | | | | | |
|---|---|---|---|---|---|---|
| | **A** | **B** | **C** | **D** | **E** | **F** |
| Bumetanide | 0.5 | 0.5 | 0.5 | 0.9 | 0.9 | 0.9 |
| KOH [10%] | q. s. to pH 7.0 | | | | | |
| K₂HPO₄ | 0 | 0.5 | 1.0 | 0 | 0.5 | 1.0 |
| Aqua purificata | Ad 100 | | | | | |

The stability of these formulations at storage temperatures of 25°C and 4°C was tested. The results are summarized in Table 8.

**Table 8**

| **Formulation** | **Storage temperature** | |
|---|---|---|
| | **25°C** | **4°C** |
| A | Stable | Unstable |
| B | Stable | Unstable |
| C | Unstable | Unstable |
| D | Stable | Unstable |
| E | Stable | Unstable |
| F | Unstable | Unstable |

### Comparative Example B

Intranasal formulations were prepared in the same as in Example 2 on the basis of the recipes shown in Table 9.

**Table 9**

| | **Wt.%** | | | |
|---|---|---|---|---|
| | **A** | **B** | **C** | **D** |
| Bumetanide | 0.5 | 0.5 | 0.9 | 0.9 |
| KOH [10%] | q. s. to pH 7.0 | | | |
| Ammonium acetate | 0.5 | 1.0 | 0.5 | 1.0 |
| Aqua purificata | Ad 100 | | | |

The stability of these formulations at storage temperatures of 25°C and 4°C was tested. The results are summarized in Table 10.

**Table 10**

| **Formulation** | **Storage temperature** | |
|---|---|---|
| | **25°C** | **4°C** |
| A | Unstable | Unstable |
| B | Unstable | Unstable |
| C | Unstable | Unstable |
| D | Unstable | Unstable |

### Example 5

Intranasal formulations containing different bumetanide salts were prepared in the same as in Example 2 on the basis of the recipes shown in Table 11.

**Table 11**

| | **Wt. %** | | | |
|---|---|---|---|---|
| | **1** | **2** | **3** | **4** |
| Bumetanide | 1.11 | 1.11 | 1.11 | 1.11 |
| Polysorbate 80 | 11 | 11 | 8 | 8 |
| NaOH [10%] | q. s. to pH 7.0 | | 0 | 0 |
| KOH [10%] | 0 | 0 | q. s. to pH 7.0 | |
| Na₂HPO₄ | 0.5 | 1.0 | 0 | 0 |
| K₂HPO₄ | 0 | 0 | 0.5 | 1.0 |
| Aqua purificata | Ad 100 | | | |

The stability of these formulations at storage temperatures of 25°C and 4°C was tested. The results are summarized in Table 12.

**Table 12**

| **Formulation** | **Storage temperature** | |
|---|---|---|
| | **25°C** | **4°C** |
| 1 | Stable | Stable |
| 2 | Stable | Stable |
| 3 | Stable | Stable |
| 4 | Stable | Stable |

### Example 6

Intranasal formulations containing different bumetanide salts were prepared in the same as in Example 2 on the basis of the recipes shown in Table 13.

**Table 11**

| | **Wt. %** | | | |
|---|---|---|---|---|
| | **1** | **2** | **3** | **4** |
| Bumetanide | 1.11 | 1.11 | 1.11 | 1.11 |
| Polysorbate 80 | 11 | 11 | 11 | 11 |
| NaOH [10%] | q. s. to pH 7.0 | | 0 | 0 |
| KOH [10%] | 0 | 0 | q. s. to pH 7.0 | |
| Ammonium acetate | 0.5 | 1.0 | 0.5 1.0 | |
| Aqua purificata | Ad 100 | | | |

The stability of these formulations at storage temperatures of 25°C and 4°C was tested. The results are summarized in Table 14.

**Table 14**

| **Formulation** | **Storage temperature** | |
|---|---|---|
| | **25°C** | **4°C** |
| 1 | Stable | Stable |
| 2 | Stable | Stable |
| 3 | Stable | Stable |
| 4 | Stable | Stable |

### Example 7

Intranasal formulations containing different bumetanide salts were prepared in the same way as in Example 2 on the basis of the recipes shown in Table 15.

**Table 15**

| | **Wt.%** | |
|---|---|---|
| | **1** | **2** |
| Bumetanide | 2.2 | 1.1 |
| Polysorbate 80 | 11.0 | 11.0 |
| NH₃ (25%) | q.s. to pH 7.0 | |
| LiOH (10%) | | q.s. to pH 7.0 |
| Ammonium acetate | 0.5 % | 0.5% |
| Aqua purificata | Ad 100 | |

The stability of these formulations at storage temperatures of 25°C and 4°C was tested. The results are summarized in Table 16.

**Table 16**

| **Formulation** | **Storage temperature** | |
|---|---|---|
| | **25°C** | **4°C** |
| 1 | Stable | Stable |
| 2 | Stable | Stable |

### Example 8

Intranasal formulations containing different thickeners were prepared in the same way as in Example 2 on the basis of the recipes shown in Table 16.

**Table 16**

| | **Wt.%** | | | | | |
|---|---|---|---|---|---|---|
| | **1** | **2** | **3** | **4** | **5** | **6** |
| Bumetanide | 2.2 | | | | | |
| Polysorbate 80 | 11.0 | | | | | |
| Alginic acid (high G) | 0.3 | | | | | |
| Sodium alginate (high G) | | 0.3 | | | | |
| HPMC | | | 1.0 | | | |
| Pectin | | | | 1.0 | | |
| Vivapur^{®} MCG ¹ | | | | | 1.0 | |
| Gellan | | | | | | 0.5 |
| NH₃ [25%] | q. s. to pH 7.0 | | | | | |
| Ammonium acetate | 0.50 | | | | | |
| Aqua purificata | Ad 100 | | | | | |

| | | | | | | |
|---|---|---|---|---|---|---|
| ¹ Co-processed composite consisting of microcrystalline cellulose and sodium carboxymethyl cellulose | | | | | | |

The stability of these formulations at storage temperatures of 25°C and 4°C was tested. The results are summarized in Table 17

**Table 17**

| **Formulation** | **Storage temperature** | |
|---|---|---|
| | **25°C** | **4°C** |
| 1 | Stable | Stable |
| 2 | Stable | Stable |
| 3 | Stable | Stable |
| 4 | Stable | Stable |
| 5 | Stable | Stable |
| 6 | Stable | Stable |

### Example 9

Intranasal formulations containing different preservatives were prepared in the same way as in Example 2 on the basis of the recipes shown in Table 18.

**Table 18**

| | **Wt.%** | | |
|---|---|---|---|
| | **1** | **2** | **3** |
| Bumetanide | 2.2 | | |
| Polysorbate 80 | 11.0 | | |
| Potassium sorbate | 0.2 | 1 | |
| Butyl p-hydroxybenzoate | | | 0.02 |
| NH3 [25%] | q. s. to pH 7.0 | | |
| Ammonium acetate | 0.50 | | |
| Aqua purificata | Ad 100 | | |

The stability of these formulations with regard to microbiological stability was tested against common pathogens. All three formulations reduced strains of *S. viridans, Neisseria mucosa, C. diphteria, S.epidermis* and *S.aureus* from 1000 to <10 colony forming units (cfu) within 24 hours.

The acceptability of these formulations with regard to nasal application was tested in volunteers. The results are summarized in table 19.

**Table 19**

| **Formulation** | **Tolerability of nasal application** |
|---|---|
| 1 | tolerable |
| 2 | tolerable |
| 3 | tolerable |
| 4 | Moderately tolerable |

### Example 10

The diuretic effect of an intranasal formulation according to the present invention was compared with the diuretic effect of a 1 mg bumetanide tablet and the diuretic effect of a placebo tablet (control) in post prandial state (after a meal). The diuretic effect was tested in healthy volunteers (without edema). The volunteers were asked to replenish their loss of urine by drinking the same amount of aqueous beverage.

The intranasal formulation was prepared in the same way as in Example 2 on the basis of the recipe shown in Table 20.

**Table 20**

| | **Wt.%** |
|---|---|
| Bumetanide | 2.2 |
| Polysorbate 80 | 11 |
| Ammonium acetate | 0.5 |
| NH3 [25%] | q. s. to pH 7.0 |
| Potassium sorbate | 0.20 |
| Aqua purificata | Ad 100 |

The intranasal formulation was administered by means of a spraying device that delivered a dose of approximately 50 µl (1 mg bumetanide). The results of the test are summarized in Table 21.

**Table 21**

| **Time period after administration** | **Cumulative urine production (in ml)** | | |
|---|---|---|---|
| | **Control** | **Oral** | **Intranasal** |
| 1 hour | 52 | 80 | 700 |
| 2 hours | 210 | 200 | 1000 |
| 3 hours | 334 | 500 | 1500 |
| 4 hours | 410 | 1000 | 1620 |
| 5 hours | 486 | 1500 | 1900 |
| 6 hours | 571 | 1800 | 2100 |

## Claims

1. An aqueous bumetanide-containing liquid comprising:
• 0.3-5 wt.% bumetanide;
• 2-30 wt.% of a non-ionic surfactant having a HLB of more than 10; and
• 65-94 wt.% water;
wherein the liquid has a pH at 25°C in the range of 5-9 and wherein each of the bumetanide and the non-ionic surfactant is dissolved and/or contained in colloidal particles having a diameter of less than 50 nm.

2. The aqueous bumetanide-containing liquid according to claim 1, wherein the non-ionic surfactant has a HLB in the range of 10-20, preferably in the range of 12-19, more preferably in the range of 14-18.

3. The aqueous bumetanide-containing liquid according to any one of the preceding claims, wherein the liquid comprises at least 4 wt.%, preferably at least 5 wt.%, most preferably at least 6 wt.% of the non-ionic surfactant.

4. The aqueous bumetanide-containing liquid according to any one of the preceding claims, wherein the non-ionic surfactant is selected from polyoxyethylene sorbitan esters of fatty acids, glycerol polyethylene glycol hydroxyl-fatty acid, polyethyleneglycol esters of fatty acids, poloxamers and combinations thereof.

5. The aqueous bumetanide-containing liquid according to any one of the preceding claims, wherein the liquid has a pH at 25°C in the range of 6 - 8.

6. The aqueous bumetanide-containing liquid according to any one of the preceding claims, wherein the liquid contains sodium, potassium, lithium, ammonium and/or arginine in a combined molar concentration that equals 50-150% of the molar concentration of bumetanide.

7. The aqueous bumetanide-containing liquid according to any one of the preceding claims, wherein the liquid comprises 0.5-4 wt.%, preferably 0.7-3 wt.% more preferably, 1.0-2.0 wt.% of bumetanide.

8. The aqueous bumetanide-containing liquid according to any one of the preceding claims, wherein the bumetanide is fully dissolved in the aqueous liquid.

9. The aqueous bumetanide-containing liquid according to any one of the preceding claims, wherein the non-ionic surfactant is fully dissolved in the aqueous liquid.

10. The aqueous bumetanide-containing liquid according to claim 9, wherein the liquid comprises a buffer having a buffering pH in the range of 6 to 8.

11. The aqueous bumetanide-containing liquid according to any one of the preceding claims, wherein the liquid has a viscosity at 20°C and a shear rate of 16 s⁻¹ of not more than 200 mPa.s, preferably less than 100 mPa.s, more preferably 2-60 mPa.s.

12. A spraying device comprising
• a container holding the aqueous bumetanide-containing liquid as defined in any one of claims 1-11,
• a nozzle in fluid connection with the container, and
• a metered dose pump configured to create a flow of the aqueous bumetanide-containing liquid through the nozzle upon activation.

13. An aqueous bumetanide-containing liquid as defined in any one of claims 1-11 for use in medical treatment, said treatment comprising transmucosal administration of the aqueous bumetanide-containing liquid.

14. The aqueous bumetanide-containing liquid for use according to claim 13, wherein the liquid is administered intranasally.

15. The aqueous bumetanide-containing liquid for use according to claim 13 or 14, wherein the liquid is administered to deliver bumetanide in a dose of 0.5 to 5 mg.

## Patentansprüche

1. Wässrige Bumetanid-enthaltende Flüssigkeit, enthaltend:
• 0,3-5 Gew.-% Bumetanid;
• 2-30 Gew.-% eines nichtionischen Tensids, das ein HLB von mehr als 10 aufweist; und
• 65-94 Gew.-% Wasser;
wobei die Flüssigkeit bei 25 °C einen pH im Bereich von 5-9 aufweist und wobei jedes von dem Bumetanid und dem nichtionischen Tensid gelöst ist und/oder in kolloidalen Teilchen enthalten ist, die einen Durchmesser von weniger als 50 nm aufweisen.

2. Wässrige Bumetanid-enthaltende Flüssigkeit nach Anspruch 1, wobei das nichtionische Tensid einen HLB im Bereich von 10-20 aufweist, bevorzugt im Bereich von 12-19, mehr bevorzugt im Bereich von 14-18.

3. Wässrige Bumetanid-enthaltende Flüssigkeit nach einem der vorhergehenden Ansprüche, wobei die Flüssigkeit zumindest 4 Gew.-%, bevorzugt zumindest 5 Gew.-%, am meisten bevorzugt zumindest 6 Gew.-% des nichtionischen Tensids enthält.

4. Wässrige Bumetanid-enthaltende Flüssigkeit nach einem der vorhergehenden Ansprüche, wobei das nichtionische Tensid ausgewählt ist aus Polyoxyethylensorbitanestern von Fettsäuren, Glycerolpolyethylenglykol-Hydroxyfettsäuren, Polyethylenglykolestern von Fettsäuren, Poloxameren und Kombinationen davon.

5. Wässrige Bumetanid-enthaltende Flüssigkeit nach einem der vorhergehenden Ansprüche, wobei die Flüssigkeit bei 25 °C einen pH im Bereich von 6-8 aufweist.

6. Wässrige Bumetanid-enthaltende Flüssigkeit nach einem der vorhergehenden Ansprüche, wobei die Flüssigkeit Natrium, Kalium, Lithium, Ammonium und/oder Arginin in einer kombinierten molaren Konzentration enthält, die 50-150 % der molaren Konzentration von Bumetanid entspricht.

7. Wässrige Bumetanid-enthaltende Flüssigkeit nach einem der vorhergehenden Ansprüche, wobei die Flüssigkeit 0,5-4 Gew.-%, bevorzugt 0,7-3 Gew.-%, mehr bevorzugt 1,0-2,0 Gew.-% von Bumetanid enthält.

8. Wässrige Bumetanid-enthaltende Flüssigkeit nach einem der vorhergehenden Ansprüche, wobei das Bumetanid vollständig in der wässrigen Flüssigkeit gelöst ist.

9. Wässrige Bumetanid-enthaltende Flüssigkeit nach einem der vorhergehenden Ansprüche, wobei das nichtionische Tensid vollständig in der wässrigen Flüssigkeit gelöst ist.

10. Wässrige Bumetanid-enthaltende Flüssigkeit nach Anspruch 9, wobei die Flüssigkeit einen Puffer enthält, der einen Puffer-pH im Bereich von 6-8 aufweist.

11. Wässrige Bumetanid-enthaltende Flüssigkeit nach einem der vorhergehenden Ansprüche, wobei die Flüssigkeit bei 20 °C und einer Schergeschwindigkeit von 16 s⁻¹ eine Viskosität von nicht mehr als 200 mPa·s, bevorzugt weniger als 100 mPa·s, mehr bevorzugt 2-60 mPa·s aufweist.

12. Sprühvorrichtung umfassend
• einen Behälter, der die wässrige Bumetanid-enthaltende Flüssigkeit wie in einem der Ansprüche 1-11 definiert, enthält,
• eine Düse in Fluid-Verbindung mit dem Behälter, und
• eine Dosierpumpe, konfiguriert um bei Aktivierung einen Fluss der wässrigen Bumetanid-enthaltenden Flüssigkeit durch die Düse zu erzeugen.

13. Wässrige Bumetanid-enthaltende Flüssigkeit wie in einem der Ansprüche 1-11 definiert, für die Verwendung in einer medizinischen Behandlung, wobei die Behandlung die transmukosale Verabreichung der wässrigen Bumetanid-enthaltenden Flüssigkeit umfasst.

14. Wässrige Bumetanid-enthaltende Flüssigkeit für die Verwendung nach Anspruch 13, wobei die Flüssigkeit intranasal verabreicht wird.

15. Wässrige Bumetanid-enthaltende Flüssigkeit für die Verwendung nach Anspruch 13 oder 14, wobei die Flüssigkeit verabreicht wird, um Bumetanid in einer Dosis von 0,5-5 mg abzugeben.

## Revendications

1. Liquide aqueux contenant du bumétanide comprenant :
• de 0,3 à 5 % en poids de bumétanide ;
• de 2 à 30 % en poids d'un tensioactif non ionique présentant une HLB de plus de 10 ; et
• de 65 à 94 % en poids d'eau ;
dans lequel le liquide présente un pH à 25 °C dans la plage de 5 à 9 et dans lequel chacun du bumétanide et du tensioactif non ionique est dissous et/ou contenu dans des particules colloïdales présentant un diamètre de moins de 50 nm.

2. Liquide aqueux contenant du bumétanide selon la revendication 1, dans lequel le tensioactif non ionique présente une HLB dans la plage de 10 à 20, de préférence dans la plage de 12 à 19, de préférence encore dans la plage de 14 à 18.

3. Liquide aqueux contenant du bumétanide selon l'une quelconque des revendications précédentes, dans lequel le liquide comprend au moins 4 % en poids, de préférence au moins 5 % en poids, de manière préférée entre toutes au moins 6 % en poids du tensioactif non ionique.

4. Liquide aqueux contenant du bumétanide selon l'une quelconque des revendications précédentes, dans lequel le tensioactif non ionique est choisi parmi les esters de sorbitane polyoxyéthylène d'acides gras, l'acide gras - glycérol polyéthylène glycol hydroxyle, les esters de polyéthylène glycol d'acides gras, les poloxamères et des combinaisons de ceux-ci.

5. Liquide aqueux contenant du bumétanide selon l'une quelconque des revendications précédentes, dans lequel le liquide présente un pH à 25 °C dans la plage de 6 à 8.

6. Liquide aqueux contenant du bumétanide selon l'une quelconque des revendications précédentes, dans lequel le liquide contient du sodium, du potassium, du lithium, de l'ammonium et/ou de l'arginine à une concentration molaire combinée qui est égale à 50 à 150 % de la concentration molaire du bumétanide.

7. Liquide aqueux contenant du bumétanide selon l'une quelconque des revendications précédentes, dans lequel le liquide comprend de 0,5 à 4 % en poids, de préférence de 0,7 à 3 % en poids, de préférence encore de 1,0 à 2,0 % en poids de bumétanide.

8. Liquide aqueux contenant du bumétanide selon l'une quelconque des revendications précédentes, dans lequel le bumétanide est complètement dissous dans le liquide aqueux.

9. Liquide aqueux contenant du bumétanide selon l'une quelconque des revendications précédentes, dans lequel le tensioactif non ionique est complètement dissous dans le liquide aqueux.

10. Liquide aqueux contenant du bumétanide selon la revendication 9, dans lequel le liquide comprend un tampon présentant un pH tampon dans la plage de 6 à 8.

11. Liquide aqueux contenant du bumétanide selon l'une quelconque des revendications précédentes, dans lequel le liquide présente une viscosité à 20 °C et un taux de cisaillement de 16 s⁻¹ de pas plus de 200 mPa.s, de préférence de moins de 100 mPa.s, de préférence encore de 2 à 60 mPa.s.

12. Dispositif de pulvérisation comprenant
• un récipient contenant le liquide aqueux contenant du bumétanide tel qu'il est défini dans l'une quelconque des revendications 1 à 11,
• une buse en communication fluidique avec le récipient, et
• une pompe doseuse conçue pour créer un écoulement du liquide aqueux contenant du bumétanide à travers la buse lors de l'activation.

13. Liquide aqueux contenant du bumétanide tel qu'il est défini dans l'une quelconque des revendications 1 à 11 destiné à être utilisé dans un traitement médical, ledit traitement comprenant une administration transmuqueuse du liquide aqueux contenant du bumétanide.

14. Liquide aqueux contenant du bumétanide destiné à être utilisé selon la revendication 13, dans lequel le liquide est administré par voie intranasale.

15. Liquide aqueux contenant du bumétanide destiné à être utilisé selon la revendication 13 ou 14, dans lequel le liquide est administré pour délivrer du bumétanide en une dose de 0,5 à 5 mg.
